# EUROPÄISCHE PATENTANMELDUNG

(11) **EP 2 746 403 A1**
(43) Veröffentlichungstag der Anmeldung: **25.06.2014**
(21) Anmeldenummer: 12199152.5
(22) Anmeldetag: 21.12.2012
(51) Int. Cl.: C12Q 1/68

(54) **Verfahren zur Bestimmung des DNA-Methylierungsgrades**

(71) Anmelder: Life Science Inkubator GmbH & Co. KG, 53175 Bonn (DE)
(72) Erfinder: Die Erfindernennung liegt noch nicht vor
(74) Vertreter: von Renesse, Dorothea

(57) **Zusammenfassung**

Verfahren zur Bestimmung des normierten DNA-Methylierungsgrads eines DNA-Abschnitts, der nur an einem Genlocus vorkommt, in einer Probe genomischer DNA umfassend die Schritte:
a) Quantitative Bestimmung der Gegenwart des DNA-Abschnitts in der Probe;
b) Quantitative Bestimmung des Vorliegens von zumindest einem differentiell methylierten C eines CpG Dinukleotids innerhalb des DNA-Abschnitts und
c) Bestimmung des normierten DNA-Methylierungsgrads des DNA-Abschnitts über die in den Schritten a) und b) bestimmten Werte.

## Beschreibung

Die vorliegende Erfindung betrifft ein Verfahren zur Bestimmung des normierten DNA-Methylierungsgrades eines DNA-Abschnitts.

Das Genom eukaryotischer Zellen besitzt ein DNA-Methylierungsmuster, welches wesentlich für die Kontrolle der Genexpression ist. Die häufigste Methylierung ist die Methylierung von Cytosin zum 5-Methyl-Cytosin.

Grundsätzlich deutet eine geringe Methylierung daraufhin, dass die entsprechende genomische Region aktiv transkribiert wird, wohingegen methylierte Abschnitte zumeist inaktiv sind.

Es ist allgemein anerkannt, dass die Analyse epigenetischer Veränderungen ein diagnostisches und prognostisches Potential bei einer Vielzahl von Erkrankungen, insbesondere von Tumorerkrankungen, bietet.

WO 2010/084154 beschreibt ein Verfahren zur Bestimmung eines DNA-Methylierungsgrades eines Genoms.

Transposons, die über das gesamte Genom verteilt sind, werden analysiert und erlauben damit eine Aussage über eine genomweite Veränderung der Methylierung.

WO 2008/149237 beschreibt ein Verfahren zu Methylierungsanalyse und seine Anwendung insbesondere hinsichtlich des Septin 9-Gens. Zur Quantifizierung wurden genomische DNAs als Standards eingesetzt.

WO 2005/075671 betrifft Verfahren zur Kalibrierung und Kontrolle von Methylierungsanalysen. Hierbei wird zur Kalibrierung des Assays komplett methylierte als auch nicht methylierte DNA eingesetzt; dabei wird die vollständig methylierte DNA beispielsweise durch Behandlung mit SssI-Methylase und die nicht methylierte DNA durch WGA (whole genome amplification) hergestellt.

Dieses Kalibrationsverfahren ist weit verbreitet, entsprechende nicht-methylierte und vollständig methylierte DNA ist kommerziell erhältlich und wird häufig als Standard verwendet. Es handelt sich hierbei um positive und negative Referenzproben, auf die eine ggf. intermediäre DNA Methylierung der Messproben bezogen werden kann, um diese relativ quantifizieren zu können.

Aufgabe der vorliegenden Erfindung war es, ein Verfahren bereitzustellen, mit dem eine Normierung des Methylierungsgrades genomischer DNA spezifischer einzelner Genabschnitte, die nur auf einem Genlocus vorhanden sind, erfolgen kann, bereitzustellen, das auf die Verwendung externer Standards verzichten kann.

Gelöst wird die Aufgabe durch ein Verfahren zur Bestimmung des normierten DNA-Methylierungsgrades eines DNA-Abschnitts, der nur an einem Genlocus vorkommt, in einer Probe genomischer DNA umfassend die Schritte:
a) Quantitative Bestimmung der Gegenwart des DNA-Abschnitts in der Probe;
b) Quantitative Bestimmung des Vorliegens von zumindest einem differentiell methylierten C eines CpG-Dinukleotids innerhalb des DNA-Abschnitts und
c) Bestimmung des normierten DNA-Methylierungsgrads des DNA-Abschnitts über die in den Schritten a) und b) bestimmten Werte.

Dabei können Schritt a) und b) in beliebiger Reihenfolge oder auch gleichzeitig erfolgen.

Erfindungsgemäß wird also eine Probe genomischer DNA eingesetzt. Die Probe genomischer DNA kann grundsätzlich aus unterschiedlichen Quellen stammen, beispielsweise durch Aufreinigung aus Zellproben erhalten werden. Geeignete Zellproben könnten beispielsweise im Rahmen einer Biopsie gewonnen werden; es kann sich jedoch auch um Blutproben, Speichelproben, Urinproben oder ähnliches handeln.

In einem Schritt erfolgt nun die quantitative Bestimmung der Gegenwart des DNA-Abschnitts in der Probe. Zusätzlich erfolgt die quantitative Bestimmung des Vorliegens von zumindest einem differentiell methylierten C eines CpG-Dinukleotids innerhalb des DNA-Abschnitts.

Unter "quantitativer Bestimmung" ist eine Detektion des Vorhandenseins zu verstehen. Dabei soll nicht eine bloße qualitative Detektion erfolgen, d.h. die Frage beantwortet werden, ob ein DNA-Abschnitt vorhanden ist, sondern dieses Vorhandensein auch quantifiziert werden (zum Beispiel durch Mengenangaben, Anzahlen von Kopien und dergleichen). Dem Fachmann sind verschiedene Verfahren bekannt, um eine solche quantitative Bestimmung durchzuführen. In einer bevorzugten Ausführungsform erfolgt diese quantitative Bestimmung über eine Amplifikation mit anschließender Messung der Menge des erzeugten Amplifikats. In einer bevorzugteren Ausführungsform ist die Amplifikation eine PCR. In einer weiteren noch bevorzugteren Ausführungsform erfolgt die quantitative Bestimmung mittels real time PCR durch Bestimmung des CT-Werts.

In einer weiteren Ausführungsform erfolgt die quantitative Bestimmung über die Hybridisierung einer markierten Sonde (z.B. Nukleinsäuresonde) mit anschließender Bestimmung der Höhe des durch die Markierung (direkt oder indirekt) erzeugten Signals. In einer weiteren Ausführungsform wird eine in situ Hybridisierung (z.B. FISH) durchgeführt mit anschließender Bestimmung der Höhe des durch die Markierung erzeugten Signals. Weitere Verfahren für solche quantitative Bestimmungen sind die Detektion mit 5-methyl-Cytosin-spezifischen Antikörpern, oder der indirekte Nachweis von an methylierte DNA bindenden Faktoren mittels spezifischer Antikörper.

Unter "differentiell methyliertes C eines CpG-Dinukleotids" wird vorliegend der in verschiedenen möglichen Ausprägungsformen vorliegende Methylierungszustand des betreffenden Cytosins verstanden. Dieser kann entweder methyliert sein, d.h. dieses Cytosin liegt als ^{5m}C vor, oder er kann unmethyliert (oder demethyliert) sein, d.h. das betrachtete Cytosin weist keine 5-Methylgruppe auf. Differentiell methyliert bezieht sich also nur auf die Möglichkeit, methyliert oder unmethyliert vorliegen zu können.

Die differentielle Methylierung ergibt sich aus der Betrachtung einer Vielzahl übereinstimmender DNA-Abschnitte in der Probe. Beispielsweise könnte die Probe genomischer DNA aus einer Probe mit 10.000 Zellen stammen. Während jedes einzelne Cytosin nur eine eindeutige Methylierung oder Nicht-Methylierung haben kann, kann sich der Grad der differentiellen Methylierung der Gesamtprobe unterscheiden, z.B. könnte ein Cytosin im DNA-Abschnitt in der Probe zur Hälfte methyliert und zur Hälfte nicht-methyliert vorliegen in einer anderen Probe könnte der gleiche DNA-Abschnitt zu 90% methyliert vorliegen.

"Ein DNA-Abschnitt der nur an einem Genlocus vorkommt" bedeutet, dass es sich um einen Bereich aus dem Genom handelt, der nur einmal vorkommt. Ein DNA-Abschnitt muss daher eine gewisse Länge aufweisen, um genügend Sequenzspezifität aufzuweisen, um ihn von anderen Genabschnitten zu unterscheiden. Auf der anderen Seite darf sich der Genabschnitt nicht auf verschiedenen Chromosomen oder verschiedenen Loci eines Chromosoms wiederholen. Bevorzugt hat ein DNA-Abschnitt eine Länge von mindestens 100 Basenpaaren, mehr bevorzugt mindestens 1.000 oder 10.000 Basenpaaren; bevorzugt kürzer als 2.000.000 oder 1.000.000 Basenpaare oder 100.000 Basenpaare.

Ein Genlocus ist die physische Position eines Gens im Genom. Besteht das Genom aus mehreren Chromosomen, ist der Genlocus der Ort auf dem Chromosom, in dem sich das Gen befindet. Verschiedene Ausprägungen oder Varianten dieses Gens werden als Allele bezeichnet, die sich alle an der gleichen Stelle auf dem Chromosom, nämlich dem Genlocus befinden.

Typischerweise liegt die Länge eines erfindungsgemäßen DNA-Abschnitts bei nicht mehr als 2,5 Mio. Basenpaaren, bevorzugt bei ≤ 1 Mio. Basenpaaren oder ≤ 500.000 Basenpaaren.

Das Genom umfasst Gene und intergenische Bereiche. "Gene" umfasst Bereiche, die durch Transkription in einzelsträngige mRNA überführt werden und zusätzliche DNA-Abschnitte, die regulatorische Funktion haben, beispielsweise Promotoren und Enhancer. Die durch Transkription abgelesenen Bereiche umfassen wiederum Exons und Introns, wobei die Introns während der Prozessierung entfernt werden. Grundsätzlich können auch Exons und Introns weitere regulatorische Elemente umfassen.

Der erfindungsgemäß verwendete Begriff "DNA-Abschnitt" umfasst somit sowohl intergenische Bereiche als auch Gene und bei Genen sowohl regulatorische Bereiche als auch Introns und Exons.

Erfindungsgemäß entscheidend ist nur, dass der DNA-Abschnitt nur an einem Genlocus vorkommt, um eine spezifische Aussage über den Methylierungsgrad dieses DNA-Abschnitts treffen zu können.

Kommt ein DNA-Abschnitt innerhalb des Genoms an verschiedenen Stellen vor, ergeben sich Methylierungsgrade, die nicht für den DNA-Abschnitt spezifisch sind, sondern gemittelte Werte darstellen, deren Aussagekraft stark vermindert ist.

So könnte beispielsweise ein DNA-Abschnitt, der im Genom zweimal vorkommt in einem der Bereiche stärker und im anderen schwächer methyliert sein. Auf Grund der Bildung des Mittelwertes würde der sich daraus ergebende Methylierungsgrad unverändert erscheinen und hätte daher keine Relevanz.

Beispiele für erfindungsgemäß verwendete DNA-Abschnitte sind insbesondere Housekeeping-Gene, zelltypspezifisch exprimierte Gene, DNA-Abschnitte, die für eine micro-RNA kodieren, DNA-Abschnitte, die für Nicht-Protein kodierende RNA kodieren sowie nicht-kodierende DNA.

Beispiele für bevorzugte DNA-Abschnitte sind z.B. die Gene

RASSF1, GSTP1, ZIC4, CD44, CDKN1A, ESR1, PLAU, RARB, SFN, TNFRSF6, TSPY, ARHI, bcl-2, BRCA1, CDKN2C, GADD45A, MTAP, PGR, SLC26A4, SPARC, SYK, TJP2, UCHL1, WIT-1, PAK6, RAD50, TLX3, PIR51, MAP2K5, INSR, FBN1, SEPT9 und GG2-1.

Erfindungsgemäß wird innerhalb eines DNA-Abschnitts gemessen, d.h. dass mindestens eine differentiell methylierte C wird relativ zum Vorkommen des DNA-Abschnitts in der Probe gemessen und bezieht sich damit auf den DNA-Abschnitt selbst in der Normierung. Damit unterscheidet sich das Verfahren wesentlich von Verfahren des Standes der Technik, bei denen eine Normierung auf einen DNA-Abschnitt bezogen wird, der sich beispielsweise auf einem Plasmid befindet oder im selben Genom, aber auf einem anderen Chromosom oder auf dem gleichen Chromosom, aber an einem anderen Genlocus.

Ein bevorzugtes Verfahren zur Bestimmung des Vorliegens einer differentiellen Methylierung eines Cytosins basiert auf der Bisulfitierung von DNA mit anschließender Analyse der erzeugten bisulfitierten DNA.

Um von isolierter DNA zu Bisulfit-konvertierter DNA zu gelangen, wird diese durch eine dem Fachmann hinlänglich bekannte Bisulfitierungsreaktion umgewandelt. Hierbei werden die unmethylierten Cytosine der DNA durch das Bisulfit in Uracil umgewandelt. Als Folge der Umwandlung können verschiedene Varianten einer umgewandelten Nukleinsäure vorliegen, die von der Anzahl der unmethylierten Cytosine dieser Nukleinsäure abhängen. Eine Nukleinsäure, die beispielsweise 2 Cytosine enthält kann demnach, je nach Methylierungszustand dieser Cytosine, zu 4 unterschiedlichen Varianten nach der Bisulfitierung führen, da entweder keines, das erste, das zweite, oder beide der Cytosine unmethyliert vorliegen kann/können und in Uracil umgewandelt wird/werden. Diese unterschiedlichen Varianten können in einer bevorzugten Ausführungsform mittels spezifischer Primer oder Primerpaare detektiert werden.

An die Umwandlung kann zunächst sich in einer Ausführungsform ein weiterer Schritt der Aufreinigung der bisulfitierten DNA anschließen. In einer weiteren Ausführungsform wird die Bisulfitierung der DNA als weiterer Schritt von dem Verfahren der Erfindung umfasst, als Bestandteil der quantitativen Bestimmung des Vorliegens eines differentiell methylierten Cytosins eines CpG Dinukleotids.

In einer weiteren, besonders bevorzugten Ausführungsform umfasst Schritt a) die Amplifikation des DNA-Abschnitts mit zumindest einem Primerpaar, das spezifisch für den DNA-Abschnitt ist, wobei die Primer bevorzugt keine möglicherweise differentiell methylierte Position des DNA-Abschnitts umfassen, d.h. kein C in einem CpG-Dinukleotid. Weiterhin umfasst Schritt b) die Amplifikation der bisulfitierten DNA mit zumindest einem Primerpaar, das spezifisch für den DNA-Abschnitt ist (welcher in Schritt a) bestimmt wurde) und das zumindest einen Primer umfasst, der zumindest eine differentiell methylierte Position des DNA-Abschnitts umfasst, d.h. zwischen zumindest einem C eines methylierten CpG und zumindest einem U/T eines bisulfitierten unmethylierten CpG diskriminieren kann.

Grundsätzlich kann auch Schritt a) mit bisulfitierter DNA durchgeführt werden, da dies nicht zu einer Veränderung der DNA-Sequenz im amplifizierten Bereich führen kann, wenn keine möglicherweise differentiell methylierte Position des DNA-Abschnitts umfasst wird.

In anderen Worten kann für die Bestimmung in Schritt a) die DNA entweder nicht-bisulfitiert (also z.B. direkt nach der Isolation aus einer Probe) oder bereits bisulfitiert vorliegen. Im ersten Fall können beliebige für das Transposon spezifische Primer zu dessen Amplifizierung verwendet werden; in letzterem Fall sollte dafür Sorge getragen werden, dass die verwendeten Primer keine differentiell methylierte Stelle umfassen (also kein Cytosin eines CpG Dinukleotids). In einer bevorzugten Ausführungsform wird in den Schritten a) und b) das gleiche Volumen von DNA eingesetzt, dabei ist die DNA bevorzugt in einem Ansatz aus einer Probe isoliert worden. Noch bevorzugter werden in Schritt a) und b) gleiche Mengen an DNA eingesetzt. Auch hierbei ist die DNA bevorzugt in einem Ansatz aus einer Probe isoliert worden.

In Schritt b), der immer mit bisulfitierter DNA ausgeführt wird, ist dies genau umgekehrt; hier muss zumindest ein Primer zumindest eine differentiell methylierte Stelle des in Schritt a) amplifizierten DNA-Abschnitts umfassen (also zumindest ein Cytosin eines CpG Dinukleotids). Es spielt hierbei keine Rolle, ob der zumindest eine Primer für die zumindest eine differentiell methylierte Stelle auf dem sense oder dem antisense Strang der DNA spezifisch ist. Über diese oder diesen Primer kann somit eine an der untersuchten gegebenen Position bestehende oder nicht bestehende Methylierung der DNA nachgewiesen werden. Wird ein Amplifikat mit Primern erhalten, die spezifisch für ein CpG sind, so lag an der betreffenden Stelle eine Methylierung der ursprünglichen DNA vor, da keine Umwandlung bei der Bisulfitierungsreaktion erfolgte. Wird ein Amplifikat mit Primern erhalten, die spezifisch für ein bisulfitiertes CpG sind, so lag an der betreffenden Stelle keine Methylierung (eine Demethylierung) der ursprünglichen DNA vor. Demgemäß kann - je nach der Art der verwendeten Primer - die Methylierung oder die Demethylierung nachgewiesen werden.

Es kann für die erfindungsgemäße Bestimmung des Methylierungsgrads entweder die Methylierung oder die Demethylierung DNA-Abschnitts bestimmt werden, da diese beiden Zustände direkt miteinander korrespondieren. Wird demnach ein CpG-spezifisches Primerpaar verwendet, so wird der DNA-Methylierungsgrad über die Methylierung bestimmt; wird ein für ein bisulfitiertes CpG spezifisches Primerpaar verwendet, so wird der DNA-Methylierungsgrad über die Demethylierung bestimmt.

In einer bevorzugten Ausführungsform ist zumindest ein Primer für zumindest eine differentiell methylierte Position des DNA-Abschnitts spezifisch; noch bevorzugter sind beide Primer für zumindest eine differentiell methylierte Position des DNA-Abschnitts spezifisch. Dies hat den Vorteil, dass eine bessere Spezifität und verbesserte Amplifikation erreicht wird.

In weiteren bevorzugten Ausführungsformen sind die verwendeten Primer für mehr als eine differentiell methylierte Position spezifisch, d.h. sie umfassen in ihrer Sequenz einen DNA-Bereich, der mehrere differentiell methylierte C umfasst. Solche Primer sind für mehr als ein Cytosin eines CpG bzw. bisulfitierten CpG spezifisch. In besonders bevorzugten Ausführungsformen sind die Primer für 2, 3, 4 oder mehr als 4 differentiell methylierte Position spezifisch.

Typischerweise sind die verwendeten Primer wenigstens 15 Nukleotide lang, bevorzugt 18, 19, 20, 21, 22, 23, 24, 25 oder mehr als 25 Nukleotide lang. Ein Primerpaar kann Primer mit verschiedener Länge umfassen. In einer bevorzugten Ausführungsform sind die Primer 18 bis 35 Nukleotide lang, in einer weiteren bevorzugten Ausführungsform sind die Primer 20-30 Nukleotide lang.

In einer bevorzugten Ausführungsform sind die Primer eines Primerpaars entweder ausschließlich für zumindest ein Cytosin eines CpG Dinukleotids oder ausschließlich für zumindest ein Cytosin eines bisulfitierten CpG Dinukleotids spezifisch.

Die Primer können die zumindest eine differentiell methylierte Position an jeder Stelle umfassen, d.h. am 5'-Ende des Primeroligonukleotids, am 3'-Ende oder an jeder Position dazwischen. In einer besonders bevorzugten Ausführungsform liegt das zumindest eine, für eine differentiell methylierte Position spezifische Nukleotid am 3'-Ende des Primers. Dies hat den Vorteil einer erhöhten Spezifität.

In einer weiteren bevorzugten Ausführungsform haben die Primer eines Primerpaars einen nahezu identischen Tₘ, bevorzugt einen Tₘ, der ≤ 3° C, ≤ 2° C, ≤1° C, ≤ 0.5° C, ≤ 0.2° C oder ≤ 0.1° C voneinander abweicht.

In einer weiteren bevorzugten Ausführungsform haben die von den Primern eingeschlossenen Sequenzbereiche eine Länge von ≥ 1 und ≥ 3000 bp, bevorzugter ≥ 10 und ≤ 2000 bp, noch bevorzugter ≥ 30 und ≤ 800 bp und am bevorzugtesten ≥ 50 und ≤ 300 bp.

Der Fachmann wird erkennen, dass nicht nur ein Primerpaar eingesetzt werden kann, sondern auch eine Vielzahl derselben. In einer weiteren Ausführungsform wird das Verfahren daher mit 2, 3, 4, 5 oder mehr als 5 Primerpaaren durchgeführt, die spezifisch für einen DNA-Abschnitt sind und die jeweils zumindest einen Primer umfassen, der für zumindest ein Cytosin eines CpG Dinukleotids oder ein bisulfitiertes Cytosin eines CpG Dinukleotid spezifisch ist. Bevorzugt haben diese mehreren Primerpaare einen nahezu identischen Tm, also bevorzugt einen Tₘ, der ≤ 3° C, ≤ 2° C, ≤ 1° C, ≤ 0.5° C, ≤ 0.2° C oder ≤ 0.1° C voneinander abweicht.

Die Bestimmung des normierten DNA-Methylierungsgrads wird erfindungsgemäß aus den in Schritt a) und b) erhaltenen quantitativen Werten erhalten, beispielsweise durch Subtraktion oder - bevorzugt - durch Division, insbesondere des Wertes aus Schritt b) durch den Wert aus Schritt a).

Im Fall der Verwendung von Amplifikation zur quantitativen Bestimmung in den Schritten a) und b) kann der Methylierungsgrad über das Verhältnis der in den beiden Amplifikationsschritten (Schritt a) und b)) gebildeten Amplifikate bestimmt werden. In besonderen Ausführungsformen wird das Verhältnis über die gebildeten Mengen an Amplifikat bestimmt, bevorzugter über die Zunahme des pro Amplifikations-Zyklus gebildeten Amplifikats, noch bevorzugter über den cycle threshold (ct) Wert während einer real time PCR.

In einer Ausführungsform wird die Gesamtmenge der beiden gebildeten Amplifikate nach einer gleichen Anzahl von Amplifikationszyklen bestimmt und miteinander in Beziehung gesetzt. Für die Bestimmung der gebildeten Amplifikate sind dem Fachmann eine Reihe von unterschiedlichen Verfahren bekannt, einschließlich spektroskopischer Verfahren, der Anfärbung mittels Ethidiumbromid oder Silber und densitometrischer Bestimmung, oder der radioaktiven Markierung mit anschließender Bestimmung über, z.B., Szintillationsmessung.

In einer bevorzugten Ausführungsform erfolgt die Bestimmung der in den beiden Amplifikationsschritten gebildeten Amplifikate mit Hilfe der real time PCR während der Bildung der Amplifikate selbst. In einer weiteren bevorzugten Ausführungsform erfolgt die Bestimmung der in den beiden Amplifikationsschritten gebildeten Amplifikate simultan während einer real time PCR.

Nach der Bestimmung der in beiden Amplifikationsschritten gebildeten Amplifikate wird der Wert für das Amplifikat im zweiten Amplifikationsschritt (Schritt b)) mittels des Werts für das Amplifikat im ersten Amplifikationsschritts (Schritt a)) normiert; z.B. durch Division oder Subtraktion der ermittelten Werte.

Hierdurch wird ein normierter Methylierungsgrad bestimmt (d.h. die normierte (De)-Methylierung).

In einer Ausführungsform erfolgt die Amplifikationen der Schritte a) und b) sowie die Bestimmung der gebildeten Amplifikate mittels der real time PCR. Hierbei werden cycle threshold (ct) Werte bestimmt, sowohl für das Primerpaar, welches für zumindest eine differentiell methylierte Position des DNA-Abschnitts spezifisch ist (Schritt b), ctₘ), als auch für das Primerpaar, welches für einen nicht differentiell methylierten Bereich des DNA-Abschnitts spezifisch ist (Schritt a), ctₖ). Der ct-Wert beschreibt den Zyklus der PCR, in dem das Fluoreszenzsignal erstmals signifikant über die Hintergrundfluoreszenz ansteigt und markiert damit den Anfang der exponentiellen Phase der PCR.

Danach wird der ct-Wert aus Schritt a) vom ct-Wert aus Schritt b) abgezogen, um zum normierten Methylierungsgrad (Act) zu gelangen. Act lässt sich also berechnen als: Δct = ctₘ - ctₖ. Ist die differentiell methylierte Position komplett methyliert und es werden methylierungsspezifische Primer verwendet, wird der Wert von ctₘ und ctₖ im Wesentlichen identisch sein. Ist ein Bereich im Wesentlichen nicht methyliert, wird er erst bei einem späteren Zyklus den ct-Wert erreichen, also einen höheren ctₘ-Wert aufweisen, so dass Act positiv wird.

In Ausführungsformen der Erfindung ist es auch möglich, zunächst eine Abtrennung methylierter Bereiche vorzunehmen. Hierzu wird die Probe genomischer DNA fragmentiert. Mittels methylierungsspezifischer Antikörper werden methylierte Fragmente ausgefällt und abgetrennt. Da in diesem Fall keine methylierten Cytosine mehr vorhanden sind, kann auf die Bisulfitreaktion verzichtet werden. Es genügt, in einer Reaktion die quantitative Bestimmung der unbehandelten Probe im Schritt a) und eine quantitative Bestimmung der unbehandelten Probe im Schritt b) vorzunehmen.

Alternativ zu den genannten Bestimmungsverfahren kann auch eine methylierungsspezifische Sequenzierung erfolgen. Hierbei kann exakt ermittelt werden, in welchem Umfang eine spezifische Position methyliert oder unmethyliert ist. Das grundsätzliche Verfahren ist dem Fachmann bekannt.

Soweit die Bestimmung in den Schritten a) und b) mittels Amplifikation erfolgt, liegt bevorzugt der Abstand zwischen dem Template für die Bestimmung in Schritt a) und dem Template für die Bestimmung in Schritt b) bei nicht mehr als 1 Mio. Basenpaaren, bevorzugt bei nicht mehr als 100.000 Basenpaaren, noch mehr bevorzugt bei nicht mehr als 10.000 oder 1.000 Basenpaaren.

In einer bevorzugten Ausführungsform wird eine nested PCR verwendet. Bei einer nested PCR werden zwei PCR-Reaktionen nacheinander durchgeführt.

In der ersten PCR wird der gesuchte PCR-Abschnitt amplifiziert und in der zweiten PCR werden die so erzeugten Abschnitte für die zweite PCR verwendet, in dem Primer verwendet werden, die einen kleineren Bereich amplifizieren, d.h. "downstream" von den ersten Primern liegen. Typisch sind eine Voramplifikation von 2-30 Zyklen mit Primern, die spezifisch sind für die methylierte Zielsequenz und eine daraus erfolgende Folgeamplifikation mit Primern, die innerhalb von 1-500 Nukleotiden stromabwärts der Primerbindestellen der voramplifizierenden Primern liegen.

In anderen Ausführungsformen der Erfindung können beispielsweise gleichzeitig zwei oder mehr differentiell methylierte Bereiche eines DNA-Abschnitts bestimmt werden. Daraus lässt sich neben der spezifischen Aussage über die differentielle Methylierung eines speziellen Sequenzabschnitts des DNA-Abschnitts auch ein gemittelter Methylierungsgrad über den gesamten DNA-Abschnitt bestimmen.

In einer anderen Ausführungsform wird vor der Bestimmung in den Schritten a) und b) eine whole genome amplification durchgeführt. Whole genome amplifications eignen sich für Untersuchungen, bei denen nur geringe Mengen an DNA vorliegen. Wenn zwei Proben verglichen werden sollen, ist es erforderlich, dass beide eine effiziente Amplifikation erfahren; eventuelle Ungleichheiten der Amplifikation kann das Verfahren durch Normierung innerhalb des gleichen DNA-Abschnitts ausgleichen.

Die Behandlung von genomischer DNA mit Bisulfit ist eine effiziente und gut etablierte Methode um DNA-Methylierung zu analysieren. Dies setzt allerdings voraus, dass eine genügende DNA-Menge konvertiert wird. Bei einigen Materialien, die diagnostisch interessant sind, ist die Voraussetzung nicht sicher erfüllt. Beispiel dafür sind FFPE-Gewebe, freie zirkulierende DNA aus Körperflüssigkeiten, etc. Deswegen ist die Analyse nur der methylierten Fraktion sehr hilfreich. Dies ermöglicht die gut etablierte methylierungsspezifische Immunopräzipitation (MeDIP). Das MeDIP Verfahren basiert auf der Affinitätsreinigung von methylierter DNA unter Verwendung eines hoch spezifischen Antikörpers, der gegen 5-Methylcytosin (5-mC) gerichtet ist. Die Qualität der präzipitierten DNA ist abhängig von deren Herkunft. So tritt das Problem der genetischen Unähnlichkeit beispielsweise bei ungleich fragmentierter DNA (FFPE-Material) sowie Tumormaterial aller Arten ein. Der Einsatz präzipitierter DNA aus solchen Materialien bei der real time PCR kann das Ergebnis wegen vorexistierenden genetischen Unähnlichkeiten verfälschen. Das vorliegende Verfahren kann die eventuell vorliegende Unähnlichkeit umgehen, indem in einer real time PCR die Zielregion mit einer Region aus der nächsten Nähe des gleichen DNA-Abschnitts normiert und dadurch die Abweichung kompensiert und korrigiert wird..

Gegenstand der Erfindung ist auch ein Verfahren zur Bestimmung des relativen DNA-Methylierungsgrads umfassend die Schritte:
a) Bestimmung des Methylierungsgrads gemäß der Schritte a) bis c) nach Anspruch 1 für eine erste genomische DNA und eine zweite genomische DNA; und
b) Bestimmung des relativen DNA-Methylierungsgrads über das Verhältnis der für die erste und die zweite DNA bestimmten Methylierungsgrade.

Diese Verfahren dient insbesondere der Diagnose einer Erkrankung, die mit einer veränderten DNA-Methylierung in Verbindung gebracht wird, wobei die erste DNA eine Referenzprobe ist und die zweite DNA aus einer zu untersuchenden Probe stammt.

Dabei Verfahren kann der relative DNA-Methylierungsgrad eine Hyper- oder Hypomethylierung bedeuten.

### Figurenbeschreibung

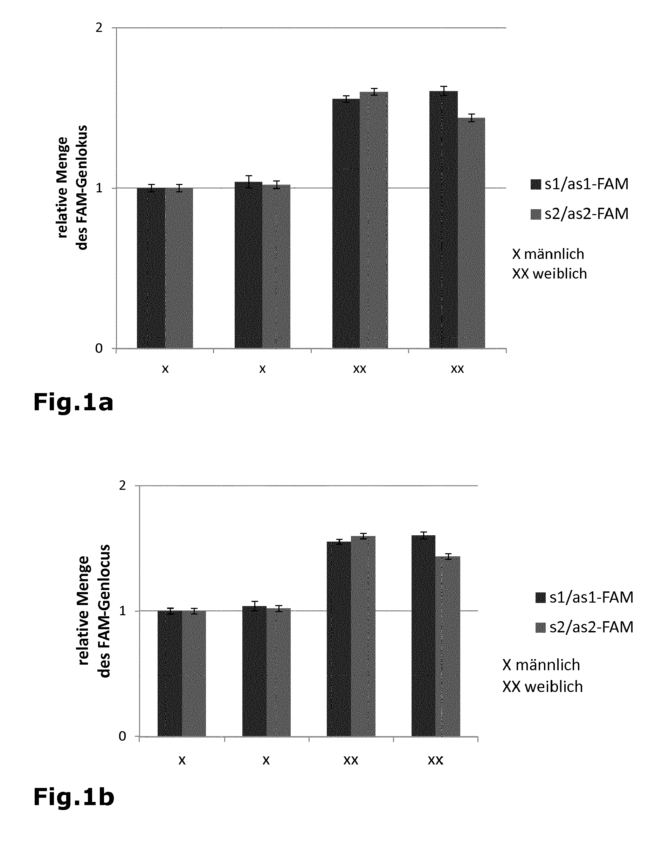

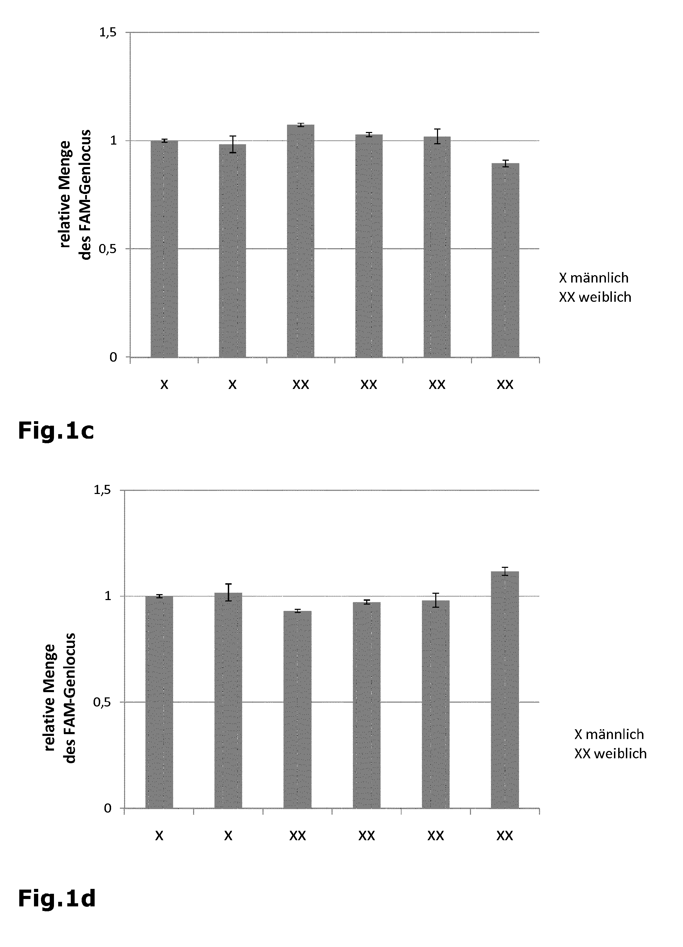

Fig.1a: Normierung des FAM-Gens auf NKP 46: Relativen Menge des X-chromosomalen FAM-Genlocus nach Quantifizierung mittels der spezifischen Primerpaare s1/as1-FAM und s2/as2-FAM und Normierung auf den NKP46-Genlocus (chr. 19) .
Fig1.b: Normierung des FAM-Gens auf GAPDH Relativen Menge des X-chromosomalen FAM-Genlocus nach Quantifizierung mittels der spezifischen Primerpaare s1/as1-FAM und s2/as2-FAM und Normierung auf den GAPDH-Genlocus (chr. 12) dargestellt.
Fig. 1c: Normierung des FAM-Genlocus s1/as1 auf den FAM-Genlocus s2/as2 Relative Menge des X-chromosomalen FAM-Genlocus nach Quantifizierung mittels des FAM spezifischen Primerpaares s1/as1 und Normierung auf den benachbarten FAM-Genlocus s2/as2-FAM
Fig.1d : Normierung des FAM-Genlocus s2/as2 auf den FAM-Genlocus s1/as1: Relative Menge des X-chromosomalen FAM-Genlocus s2/as2 nach Quantifizierung mittels des spezifischen FAM Primerpaares s2/as2 und Normierung auf den benachbarten FAM-Genlocus s1/as2 nach Quantifizierung mittels des spezifischen FAM Primerpaares s1/as1.
Fig.2a : Normierung des JAM2-Gens auf das NKP46: Relativen Menge des JAM2-Genlocus nach Quantifizierung mittels des spezifischen JAM2 Primerpaare s1/as1 und s3/as3 und Normierung auf den NKP46-Genlocus (chr. 19).
Fig.2b: Normierung des JAM2-Gen auf das GAPDH
   Relative Menge des JAM2-Genlocus nach Quantifizierung mittels des spezifischen Primerpaares s3/as3-JAM2 und s2/as2-JAM und Normierung auf den GAPDH-Genlocus (chr. 12).
Fig.2c: Normierung des JAM2-Genlocus s1/as1 auf den JAM2-Genlocus s3/as3 Relative Menge des JAM2-Genlocus nach Quantifizierung mittels des JAM2 spezifischen Primerpaares s3/as3 und Normierung auf den benachbarten JAM2-Genlocus s1/as1 nach Quantifizierung mittels des JAM2spezifischen Primerpaares s1/as1.
Fig.2d: Normierung des JAM2-Genlocus s3/as3 auf den JAM2-Genlocus s1/as1^Relative Mengen des JAM2-Genlocus nach Quantifizierung mittels des JAM2 spezifischen Primerpaares s3/as3 und Normierung auf den benachbarten JAM2-Genlocus s1/as1 nach Quantifizierung mittels des JAM2spezifischen Primerpaares s1/as1

Die Erfindung wird durch die nachfolgenden Beispiele näher erläutert.

### Beispiel 1: Fam

Als simulierte Aneuploidie wird das X-chromosomal gekoppelte FAM verwendet. FAM kommt wie das X-Chromosom in weiblicher DNA zweimal, in männlicher nur einmal vor. Es wurde mit dem konventionellen Normierungsverfahren, d. h. bezogen auf ein Housekeeping-Gen (GAPDH und NKP46) normiert sowie mit dem erfindungsgemäßen Verfahren.

Genomische DNA wurde aus Blut verschiedener gesunder weiblicher und männlicher Probanden isoliert. Hierfür wurde das QIAamp DNA-Blood-Mini-Kit (Qiagen) verwendet. 200 ng DNA wurden anhand des EpiTect Bisulfite-Kit (Qiagen) nach den Anweisungen des Herstellers konvertiert. Methylierungsspezifische Primer wurden mittels des Programmes Oligo designed. 15 ng Bisulfit-konvertierte DNA wurde für die methylierungsspezifische PCR eingesetzt. Als Optimum für die Versuche in der MS-PCR ergaben sich für das Primerpaar s1/as1-FAM 57°C und das Primerpaar s2/as2-FAM 66°C. Es wurden 10 pmol Primer per Reaktion verwendet.

### Ergebnis

Die konventionelle Normierung der FAM-Genloci auf den Genlocus des Housekeeping-Gens NKP46 ergibt, dass der FAM-Genlocus im Mittel 1,5 fach bei den weiblichen gegenüber den männlichen Proben vorhanden ist (Figur 1a).

Bei der konventionellen Normierung der FAM-Genloci auf das Housekeeping-Gen GAPDH ergibt sich im Vergleich zur Normierung auf NKP46 ein ähnliches Ergebnis, d. h. die 1,5 fache relative Menge des FAM-Genlocus bei weiblichen gegenüber männlichen Proben (Figur 1b).

Nach dem erfindungsgemäßen Verfahren ergeben sich gleiche normierte Mengen der FAM-Genloci in den weiblichen wie in den männlichen Proben sowohl bei der Normierung des DNA-Abschnitts FAM über die Positionen s1/as1 und s2/as2 (Figur 1c).

### Beispiel 2: JAM2

Als simulierte Aneuploidie wird das JAM2 Gen verwendet. Dieses liegt bei Gesunden diploid (2fach), bei Trisomie 21 triploid (3fach) vor. Das entspricht einer Aneuploidie im Verhältnis 1:1,5 von Gesundem zu Trisomie 21.

Es wurde mit dem konventionellen Normierungsverfahren, d. h. bezogen auf ein Housekeeping-Gen (GAPDH und NKP46) normiert sowie mit dem erfindungsgemäßen Verfahren.

Genomische DNA wurde aus Blut verschiedener gesunder weiblicher und männlicher Probanden isoliert. Dazu wurde eine Probe verwendet, bei der eine Trisomie 21 vorliegt und cytogenetisch bestätigt. Hierfür wurde das QIAamp DNA-Blood-Mini-Kit (Qiagen) verwendet. 200 ng DNA wurden anhand des EpiTect Bisulfite-Kit (Qiagen) nach den Anweisungen des Herstellers konvertiert. Methylierungsspezifische Primer wurden mittels des Programmes Oligo designend. 15 ng Bisulfit-konvertierte DNA wurde für die methylierungsspezifische PCR eingesetzt .Als Optimum für die Versuche in der MS-PCR ergaben sich für das Primerpaar s1/as1-JAM und das Primerpaar s3/as3-JAM mit Annealingstemperatur von 67°C. Es wurden 10 pmol Primer per Reaktion verwendet.

### Ergebnis

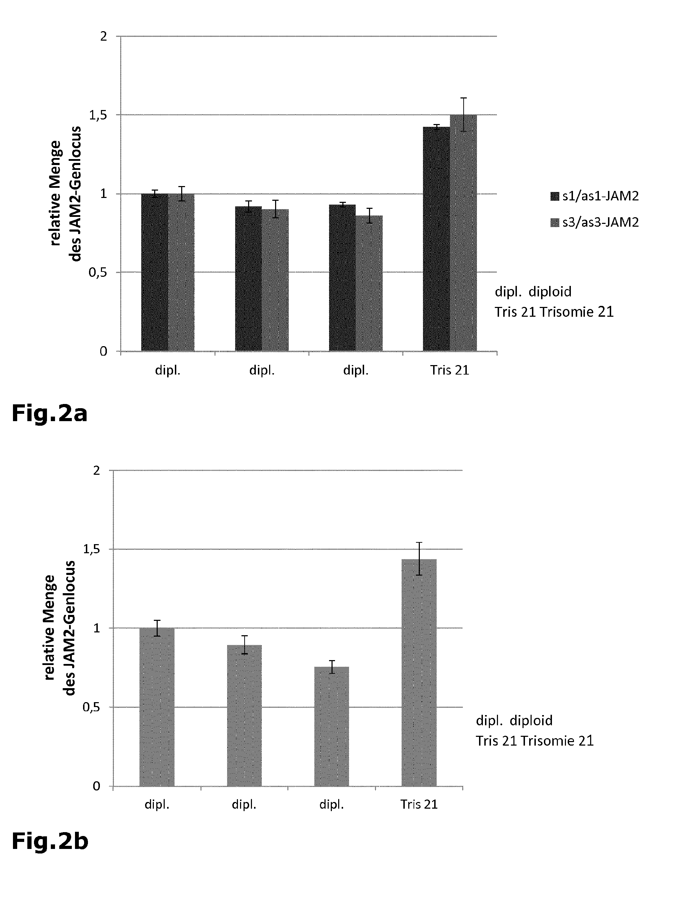

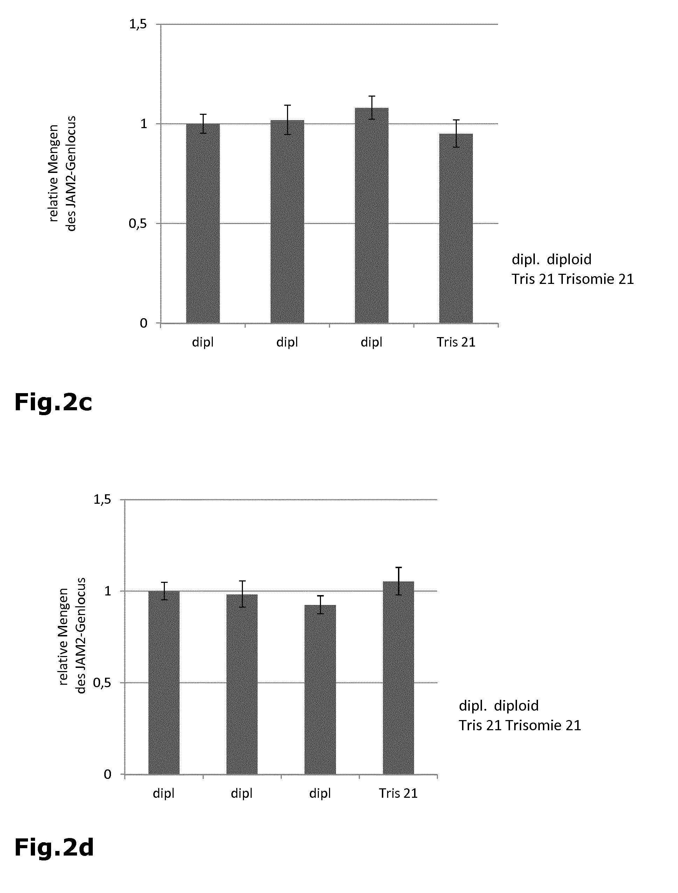

Die konventionelle Normierung von JAM2 auf die die Housekeeping-Gene GAPDH und NKP46 zeigt mindestens die 1,5 fache relative Menge bei Trisomie 21 gegenüber den "gesunden" - diploiden - Proben (Figur 2a, 2b).

Nach dem erfindungsgemäßen Verfahren ergeben sich annähernd gleiche relative Mengen der JAM2-Genloci in den "gesunden" (diploiden) wie in der Trisomie 21-Probe sowohl bei der Normierung von JAM2 s1/as1 auf JAM s3/as3 (Figur 2c).

### Beispiel 3: RASSF1

Als simulierte Proben wurden die Blasenkrebszelllinien HT1376 und Sw1710 verwendet. Die Zelllinie HT1376 hat einen Zugewinn des Chromosoms 3, in dem das RASSF1 Gen liegt und hat einen Verlust des 12p12.3-pter Bereichs, in dem das GAPDH Gen liegt (Hurst et al., Oncogene 2004; 23(12):2250-63). Die DNA Methylierung des RASSF1 5' Bereichs ist in HT1376 vergleichbar hoch wie in der Harnblasenkarzinomzelllinie Sw1710 (Neuhausen et al., Cancer Biol Ther. 2006 Aug; 5(8):993-1001.). Dazu wurde mit dem konventionellen Normierungsverfahren, d. h. bezogen auf ein Housekeeping-Gen (GAPDH und NKP46) normiert sowie mit dem erfindungsgemäßen Verfahren.

Genomische DNA wurde aus gut beschriebenen Zelllinien isoliert. Hierfür wurde das QIAamp DNA-Blood-Mini-Kit (Qiagen) verwendet. 200 ng DNA wurden anhand des EpiTect Bisulfite-Kit (Qiagen) nach den Anweisungen des Herstellers konvertiert. Methylierungsspezifische Primer wurden mittels des Programmes Oligo designend. 15 ng Bisulfit-konvertierte DNA wurde für die methylierungsspezifische PCR eingesetzt. Das Optimum des Primerpaares RASSF1 Kon1 (Kontrollprimerpaar) lag bei 54 °C und für RASSF1 MS (methylspezifisches Primerpaar) bei 58°C. Es wurden 10 pmol Primer per Reaktion verwendet.

### Konventionelle Normierung

Proben von Sw1710 und HT1376 umfassend 15 ng DNA wurden mit den Priomern s/as RASSF1in parallelen Experimenten amplifiziert. Es ergaben sich gemittelte ct-Werte von
Sw1710: 27,16
HT1376: 25,86

Zur Normierung wurden die Primer s1/as für GAPDH verwendet; die gemessenen gemittelten ct-Werte betrugen
Sw1710: 26,15
HT1376: 78,74

Die Differenz (ΔCT) zwischen dem ct-Wert für RASSF1 und der GAPDH Kontrolle betrug
Sw1710: 1,01
HT1376: -2,88

Die Zelllinie Sw1710 wurde als Standard angesehen. Die Differenz der Δct-Werte (ΔΔCt) betrug dann -3,90. Das Verhältnis des Vorkommens der methylierten Sequenz ergibt sich dann als 2^{-ΔΔt} = 14,9.

### Erfindungsgemäße Normierung

Wie bei der konventionellen Normierung wurden die ct-Werte der beiden Zellen bestimmt. Es ergaben sich die ct-Werte von
Sw1710: 27,16
HT1376: 25,86

In diesem Fall wurde aber als Kontrolle der Primer s1/as1 RASSF1 Kontrolle verwendet. Hiermit ergaben sich ct-Mittelwerte von
Sw1710: 23,7
HT1376: 24,70

Die Differenz (ΔCT) zwischen dem ct-Wert für RASSF1 und der RASSF1 Kontrolle betrug
Sw1710: 3,46
HT1376: 1,16

Die Differenz der beiden ct-Werte (ΔΔCt) betrug -2,31. Damit ergab sich eine Differenz des Vorkommens von 2^{-ΔΔct} von 4,95.

### Ergebnis

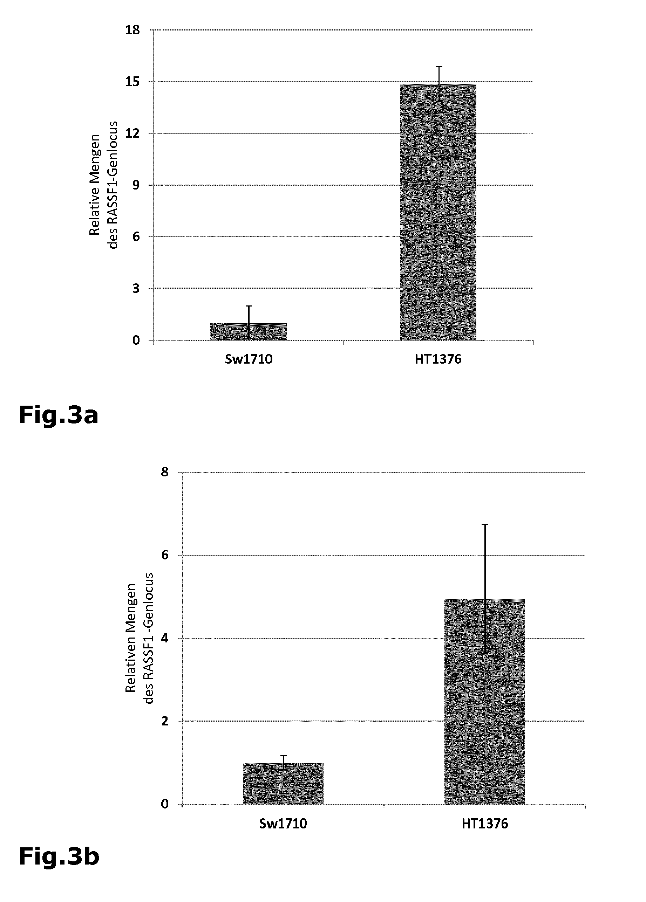

Nach der konventionellen Normierung beträgt die Methylierung von RASSF1 in der Blasenzelllinie HT1376 das 15 Fache gegenüber der in Sw1710 (Figur 3a). Nach dem erfindungsgemäßen Verfahren ist sie annähernd nur noch 5 fach so hoch (Figur 3b).

### Beispiel 4: Verwendete Primer in den Beispielen 1 bis 3

**Tabelle 1: NKP46-Primersequenzen**

| Bezeichnung | DNA-Sequenz von 5' -> 3'-Rirchtung |
|---|---|
| s1 | CACGGCCTTTCTGTAAGCTCATGGTC |
| as1 | CAGCGTGATCCCATTCCCCTTCC |

**Tabelle 2: JAM2-Primersequenzen**

| Bezeichnung | DNA-Sequenz von 5' -> 3'-Rirchtung |
|---|---|
| s1 | TCCCCTCCCGACTCTCTGCTC |
| as1 | CCAGGCAGGAAGGGTAGAGGAAC |
| s3 | ATACATCCCCGTCCCCGAG |
| as3 | CTAGAGGGCGTGAAAACCAGAC |

**Tabelle 3: GAPDH-Primersequenzen**

| Bezeichnung | DNA-Sequenz von 5' -> 3'-Rirchtung |
|---|---|
| s1 | GGGACCTTCTTGCCTTGCTCTTGCT |
| as1 | TGAGTGTGGGATGGGAGGGTGCT |

**Tabelle 4: FAM- Primersequenzen**

| Bezeichnung | DNA-Sequenz von 5' -> 3'-Rirchtung |
|---|---|
| s1 | ATGATGCTTACCCTCTCCTTGTG |
| as1 | TCTCTGCTGCTTCTGGGTCC |
| s2 | CCAGAGAGGCGGACGAAGTT |
| as2 | TTTCACGCACTCCCATTTCTG |
| s3 | GGCTCACAAAGGATGGGGG |
| as3 | TAACTTCGTCCGCCTCTCTGG |

**Tabelle 5: RASSF1-Primersequenzen**

| Bezeichnung | DNA-Sequenz von 5' -> 3'-Rirchtung |
|---|---|
| s | GCGGTCGTCGTCGTTGTGGTC |
| as | GCCCAACGAATACCAACTCCCG |
| s Kon1 | TGTGTTTAGTTTTTTAGAGTAGGATTTG |
| as Kon1 | CTCTAAACCACTACCTCTAACACATC |

**Tabelle 6: konvertierte GAPDH-Primersequenzen**

| Bezeichnung | DNA-Sequenz von 5' -> 3'-Rirchtung |
|---|---|
| s KonvPr | GGTTTTTAGTGTTTAGTGTTTAGTGT |
| as KonvPr | CCCTTTCTTTCTTTCAAAAAC |

## Patentansprüche

1. Verfahren zur Bestimmung des normierten DNA-Methylierungsgrads eines DNA-Abschnitts, der nur an einem Genlocus vorkommt, in einer Probe genomischer DNA umfassend die Schritte:
a) Quantitative Bestimmung der Gegenwart des DNA-Abschnitts in der Probe;
b) Quantitative Bestimmung des Vorliegens von zumindest einem differentiell methylierten C eines CpG Dinukleotids innerhalb des DNA-Abschnitts und
c) Bestimmung des normierten DNA-Methylierungsgrads des DNA-Abschnitts über die in den Schritten a) und b) bestimmten Werte.

2. Verfahren nach Anspruch 1, wobei der DNA-Abschnitt ausgewählt wird aus einem housekeeping-Gen, einem zelltypspezifisch exprimierten Gen, einem DNA-Abschnitt, der für eine micro-RNA codiert, einem DNA-Abschnitt, der für eine nichtproteincodierende RNA codiert und nicht-codierender DNA.

3. Verfahren nach Anspruch 1 oder 2, wobei der DNA-Abschnitt ausgewählt ist aus folgenden Genen:
RASSF1, GSTP1, ZIC4, CD44, CDKN1A, ESR1, PLAU, RARB, SFN, TNFRSF6, TSPY, ARHI, bcl-2, BRCA1, CDKN2C, GADD45A, MTAP, PGR, SLC26A4, SPARC, SYK, TJP2, UCHL1, WIT-1, PAK6, RAD50, TLX3, PIR51, MAP2K5, INSR, FBN1, SEPT9 und GG2-1.

4. Verfahren nach einem der Ansprüche 1 bis 3, wobei eine Abtrennung mittels methylierungspezifischer Antikörper erfolgt.

5. Verfahren nach einem der Ansprüche 1 bis 4, wobei der DNA-Abschnitt zur quantitativen Bestimmung einer Bisulfit-Reaktion unterzogen wird.

6. Verfahren nach Anspruch 5, wobei Real-Time PCR zur quantitativen Bestimmung verwendet wird.

7. Verfahren nach Anspruch 6, wobei der Abstand zwischen einem PCR-Template des DNA-Abschnitts für die Bestimmung gemäß a) und einem PCR-Template für die Bestimmung gemäß b) nicht mehr als 1.000.000 Basenpaare beträgt.

8. Verfahren nach einem der Ansprüche 6 bis 7, wobei für die Real-Time PCR Primer verwendet werden, deren 3'-Ende einer potentiell differentiell methylierten Position des DNA-Abschnitts entsprechen.

9. Verfahren nach einem der Ansprüche 1 bis 8, wobei die Bestimmung des normierten DNA-Methylierungsgrades über einen Quotienten oder eine Differenz der in a) und b) bestimmten Werte erfolgt.

10. Verfahren nach einem der Ansprüche 6 bis 9, wobei eine nested PCR eingesetzt wird.

11. Verfahren nach einem der Ansprüche 1 bis 10, wobei zwei potentiell differentiell methylierte Positionen eines DNA-Abschnitts bestimmt werden.

12. Verfahren nach einem der Ansprüche 1 bis 11, wobei vor der Bestimmung eine whole genome amplification durchgeführt wird.

13. Verfahren zur Bestimmung des relativen DNA-Methylierungsgrads umfassend die Schritte:
a) Bestimmung des Methylierungsgrads gemäß der Schritte a) bis c) nach Anspruch 1 für eine erste genomische DNA und eine zweite genomische DNA; und
b) Bestimmung des relativen DNA-Methylierungsgrads über das Verhältnis der für die erste und die zweite DNA bestimmten Methylierungsgrade.

14. Verfahren nach Anspruch 13 zur Diagnose einer Erkrankung, die mit einer veränderten DNA-Methylierung in Verbindung gebracht wird, wobei die erste DNA eine Referenzprobe ist und die zweite DNA aus einer zu untersuchenden Probe stammt.

15. Verfahren nach Anspruch 13 oder 14, wobei der relative DNA-Methylierungsgrad eine Hyper- oder Hypomethylierung bedeutet.
